(19) Europäisches Patentamt
European Patent Office
Office européen des brevets

(11) Veröffentlichungsnummer : **0 026 872**
**B1**

(12) # EUROPÄISCHE PATENTSCHRIFT

(45) Veröffentlichungstag der Patentschrift : **09.03.83**

(21) Anmeldenummer : **80105705.0**

(22) Anmeldetag : **23.09.80**

(51) Int. Cl.³ : **C 07 C 93/06**, C 07 D295/08, C 07 C149/42, C 07 D487/04, A 01 N 43/90, A 01 N 39/00// C07C43/225 ,(C07D487/04, 209/00, 209/00)

(54) **Pflanzenwachstumsregulierende Mittel enthaltend substituierte Alkylammoniumsalze und deren Verwendung.**

(30) Priorität : **08.10.79 DE 2940765**

(43) Veröffentlichungstag der Anmeldung : **15.04.81 Patentblatt 81/15**

(45) Bekanntmachung des Hinweises auf die Patenterteilung : **09.03.83 Patentblatt 83/10**

(84) Benannte Vertragsstaaten : **AT BE CH DE FR GB IT LI NL SE**

(56) Entgegenhaltungen :
**DE A 2 017 497**

**HELVETICA CHIMICA ACTA, vol. 54, Fasc. 1 (1971) Nr. 9-10, R. K. JOSHI et al. « Studies on Solubilization (Part I) ; Note on the synthesis of some quaternary N-(w-aryloxyalkyl) piperidinium, pyridinium, benzyldimethyl-ammonium, and trimethyl-ammonium bromides », Seiten 112-117.**

**J. ORG. CHEM. vol. 17 (1952) Washington D.C. USA R. M. HERBST et al. « The synthesis of some tertiary naphtoxyethyl-amines », Seiten 693-7.**

(73) Patentinhaber : **BASF Aktiengesellschaft**
**Carl-Bosch-Strasse 38**
**D-6700 Ludwigshafen (DE)**

(72) Erfinder : **Rentzea, Costin, Dr.**
**Neuenheimer Landstrasse 72**
**D-6900 Heidelberg (DE)**
Erfinder : **Sauter, Hubert, Dr.**
**Neckarpromenade**
**D-6800 Mannheim (DE)**
Erfinder : **Jung, Johann, Dr.**
**Hardenburgstrasse 19**
**D-6703 Limburgerhof (DE)**

**EP 0 026 872 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Pflanzenwachstumsregulierende Mittel enthaltend substituierte Alkylammoniumsalze und deren Verwendung

Die vorliegende Erfindung betrifft das Pflanzenwachstum regulierende Mittel, welche substituierte Alkylammoniumsalze enthalten, und die Verwendung dieser Verbindungen zur Regulierung des Pflanzenwachstums.

Es ist bereits bekannt, quartäre Ammoniumverbindungen, wie Chlorcholinchlorid (CCC, US-PS 3 156 544) oder 1-(β-Chlorethyl)-1,1-dimethylhydraziniumchlorid (CMH, Naturwissenschaften *55*, 217 (1968)) zur Regulierung des Pflanzenwachstums zu verwenden. Deren Wirkung ist jedoch, insbesondere bei niedrigen Aufwandmengen, nicht immer ausreichend und genügt den Anforderungen der Praxis nicht immer.

Weiterhin ist es bekannt (DE-OS 20 17 497), gewisse quartäre Phenoxyethylammoniumsalze, deren Stickstoff jedoch nicht Teil eines Heterocyclus ist, zur Beeinflussung des Pflanzenwachstums zu verwenden. Deren Wirkung ist auch, insbesondere bei niedrigen Konzentrationen, nicht immer befriedigend.

Schließlich sind in Helv. Chim. Acta *54*, 112 (1171) und J. Org. Chem. *17*, 693 (1952) Alkylammoniumsalze beschrieben, welche Arzneimittelwirkstoffe darstellen.

Gegenstand der Erfindung sind substituierte Alkylammoniumsalze der Formel I

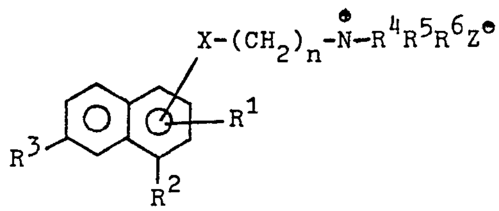

(I)

in welcher

$R^1$, $R^2$ und $R^3$ gleich oder verschieden sind und Wasserstoff, $C_{1-4}$-Alkyl oder Fluor, Chlor oder Brom bedeuten,

X Sauerstoff oder Schwefel darstellt,

n die Zahl 2, 3 oder 4 ist,

$R^4$ geradkettiges oder verzweigtes $C_{1-6}$-Alkyl, $C_{2-6}$-Alkenyl oder $C_{2-6}$-Alkinyl bedeuten, die durch Halogen, Cyan oder $C_{1-4}$-Alkoxy substituiert sein können, und $R^5$ und $R^6$ als Alkenylgruppen Teil eines gegebenenfalls durch $C_{1-2}$-Alkyl substituierten 5- oder 6-gliedrigen Heterocyclus mit 1 bis 3 Heteroatomen im Ring sind und

Z das Anion einer beliebigen nicht phytotoxischen Säure bedeutet.

Bevorzugt sind Verbindungen der Formel I, in denen der Rest $X\text{-}(CH_2)_n\text{-}N^{\oplus}R^4R^5R^6Z^{\ominus}$ sowohl in 1- als auch in 2-Stellung des Naphthalins steht und in denen X Sauerstoff, $R^1$, $R^2$ und $R^3$ Wasserstoff, Methyl oder Brom und n = 2 bedeuten.

In einer weiteren Gruppe bevorzugter Verbindungen der Formel I stehen $R^4$, $R^5$ und $R^6$ für Trimethyl-, Triethyl-, Tripropyl-, Tributylgruppen oder den Pyrrolizidinrest oder für einen am quartären Stickstoff durch $R^4$ substituierten Pyrrolidin-, Piperidin- oder Morpholinring.

Bevorzugte Reste $R^4$ sind Methyl, Ethyl, Propyl, Butyl, Allyl, Propargyl, 2-Chlorethyl, 2-Bromethyl und 2-Buten-l-yl.

Da die Wirkung der erfindungsgemäßen Verbindungen der Formel I auf das Kation zurückzuführen ist, kann das Anion $Z^{\ominus}$ aus den nicht phytotoxischen Säuren beliebig gewählt werden. Z bedeutet beispielsweise Methylsulfonat, p-Dodecylbenzolsulfonat, Sulfat, Methosulfat, Nitrat, Phosphat, lodid und insbesondere Chlorid und Bromid.

Die substituierten Alkylammoniumsalze der Formel I lassen sich herstellen, indem man

a) Verbindungen der Formel II

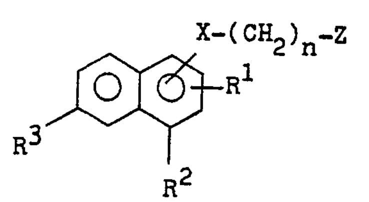

(II)

in welcher $R^1$, $R^2$, $R^3$, X, Z und n die oben angegebenen Bedeutungen haben, mit einem tertiären Amin der Formel III

$NR^4R^5R^6$ (III)

in der $R^4$, $R^5$ und $R^6$ die oben angegebenen Bedeutungen haben, oder

b) Verbindungen der Formel IV

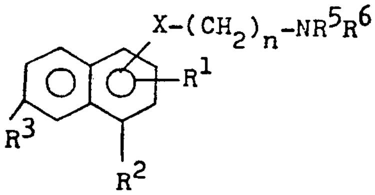

(IV)

in welcher $R^1$, $R^2$, $R^3$, $R^5$, $R^6$, X und n die oben angegebenen Bedeutungen haben, mit Alkylierungsmitteln der Formel V

$R^4$—Z (V)

in der $R^4$ und Z die oben angegebenen Bedeutungen haben, umsetzt.

Die Reaktionen a) und b) werden gegebenenfalls in Gegenwart eines Lösungs- oder Verdünnungsmittels, bei Temperaturen zwischen 20-150 °C, vorzugsweise zwischen 50 und 120 °C durchgeführt. Als bevorzugte, gegenüber den Reaktionsteilnehmern inerte Lösungs- oder Verdünnungsmittel werden z. B. aliphatische oder aromatische Kohlenwasserstoffe — wie Pentan, Cyclohexan, Benzol, Toluol, Xylol-, Halogenkohlenwasserstoffe — wie Chlorbenzol oder Dichlorbenzole-, Ketone — wie Aceton, Methylethylketon, Diethylketon und Cyclopentanon-, Ether — wie Diethylether, Dimethoxyethan, Tetrahydrofuran oder Dioxan-, Ester — wie Essigsäureethylester-, Nitrile — wie Acetonitril-, Amide — wie Dimethylformamid, Dimethylacetamid und N-Methylpyrrolidon — oder entsprechende Gemische verwendet.

Die Verbindungen der Formel II können leicht nach bekannten Verfahren hergestellt werden, beispielsweise durch Veretherung von 1- und 2-Naphtholen bzw. von 1- und 2-Thionaphtholen mit aliphatischen Dihalogeniden — wie 1,2-Dibromethan, 1,3-Dibrompropan, 1-Chlor-3-brompropan oder 1,4-Dibrombutan — bevorzugt in siedendem Methylethylketon, Diethylketon oder Cyclopentanon in Gegenwart von mindestens äquivalenten Mengen Kaliumcarbonat (vgl. Houben-Weyl, Methoden der Organischen Chemie, Band 6/3, S. 54-59, Thieme-Verlag, Stuttgart 1965).

Als tertiäre Amine der Formel III können z. B., N-Methylpyrrolidon, N-Ethylpyrrolidon, N-Allylpyrrolidon, N-Propylpyrrolidon, N-Butylpyrrolidon, N-Methylpiperidin, N-Ethylpiperidin, N-Ethylmorpholin, Chinuclidin oder Pyrrolizidin eingesetzt werden.

Die tertiären Amine der Formel IV lassen sich nach bekannten Verfahren leicht herstellen, beispielsweise nach dem Schema

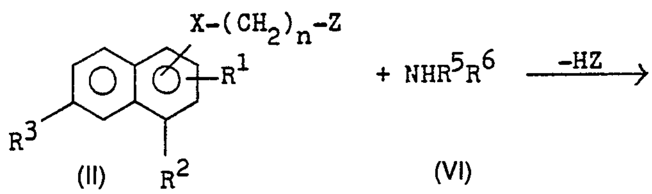


(II) (VI) (IV)

durch Alkylierung sekundärer Amine der Formel VI, in der $R^5$ und $R^6$ die oben genannten Bedeutungen haben, mit den Verbindungen der Formel II. Die Reaktionsbedingungen entsprechen hierbei denen der Umsetzung nach dem Schema II + III → I, wobei hier ein 2- bis 10-fach molarer Überschuß des Amins der Formel VI bevorzugt ist. Das bei der Umsetzung entstandene HZ kann leicht z. B. durch Behandeln des Reaktionsgemisches mit wäßrigen Alkalihydroxiden entfernt werden.

Als Amine der Formel VI können beispielsweise Pyrrolidin, 2-Methylpiperidin, 3-Methylpiperidin, 4-Methylpiperidin, Morpholin oder 2,6-Dimethylmorpholin eingesetzt werden.

Als Alkylierungsmittel der Formel V können beispielsweise Methylchlorid, Methylbromid, Methyliodid, Dimethylsulfat, Ethylchlorid, Ethyliodid, Diethylsulfat, Propylbromid, Propyliodid, Allylchlorid, Allylbromid, Butylbromid, Butylchlorid, Propargylchlorid, Chloraceton oder Chloracetonitril eingesetzt werden.

Für die Herstellung von Ausgangsverbindungen der Formeln II und IV können z. B. die folgenden Naphthole und Thionaphthole eingesetzt werden :

1-Naphthol, 2-Methyl-1-naphthol, 4-Methyl-1-napthol, 4-Chlor-1-naphthol, 4-Brom-1-naphthol, 2-Chlor-4-methyl-1-naphthol, 2-Brom-4-methyl-1-naphthol, 4-Chlor-2-methyl-1-naphthol, 4-Brom-2-methyl-1-naphthol, 2,4-Dichlor-1-naphthol, 2,4-Dimethyl-1-naphthol, 2-Naphthol, 1-Chlor-2-naphthol, 1-Brom-2-naphthol, 6-Brom-2-naphthol, 1,6-Dichlor-2-naphthol.

Die folgenden Beispiele erläutern die Herstellung der neuen Verbindungen der Formel I und der zu ihrer Herstellung nötigen Zwischenprodukte der Formeln II und IV.

Beispiel 1

a) Herstellung des Ausgangsmaterials

50,2 g (0,2 Mol) 1-Brom-2-(2-naphthyloxy)-ethan, 200 ml trockenes Dioxan und 68 g (0,8 Mol) Piperidin werden 12 Stunden bei 60 °C gerührt, abgekühlt und anschließend im Vakuum eingeengt. Der Rückstand wird zwischen 150 ml Wasser und 300 ml Ether verteilt und unter Eiskühlung mit 100 ml 50 %iger Natronlauge versetzt. Die Etherschicht wird abgetrennt, mit 100 ml Wasser gewaschen, getrocknet und im Vakuum eingeengt. Der feste Rückstand wird mit 50 ml Pentan bei +10 °C 20 Minuten gerührt, abgesaugt und mit 20 ml kaltem Pentan gewaschen. Man erhält 44 g (86,2 % d. Th.) N-2-(2-Naphthyloxy)-ethylpiperidin als farblose Kristalle vom Schmelzpunkt 49-51 °C.

b) Herstellung des Endprodukts

Die Lösung von 15,3 g (0,06 Mol) N-2-(2-Naphthyloxy)-ethyl-piperidin und 24,4 g (0,2 Mol) Allylbromid in 30 ml trockenem Dioxan und 30 ml trockenem Acetonitril wird 24 Stunden bei 60 °C gerührt. Nach Abziehen des Lösungsmittels und des überschüssigen Allylbromids im Vakuum rührt man den festen Rückstand 20 Minuten mit 50 ml trockenem Ether und nutscht anschließend den kristallinen Niederschlag ab. Man erhält 18,1 g (80 % d. Th.) N-Allyl-N-2(2-naphthyloxy)-ethyl-piperidiniumbromid als weiße Kristalle vom Schmelzpunkt 156-158 °C.

Beispiel 2

a) Herstellung des Ausgangsmaterials

Eine Mischung aus 144 g 2-Naphthol (1 Mol), 600 ml Diethylketon, 275 g (2 Mol) Kaliumcarbonat und 850 g (4,5 Mol) 1,2-Dibromethan werden 48 Stunden am Rückfluß erhitzt. Sodann destilliert man Diethylketon und überschüssiges Dibromethan im Vakuum ab und nimmt den Rückstand in 500 ml Methylenchlorid und 200 ml 10 %iger Natronlauge auf. Nach Abtrennen der wäßrigen Phase wird die organische Schicht mit 100 ml Wasser gewaschen, über Natriumsulfat getrocknet und im Vakuum eingeengt. Der feste Rückstand wird mit 100 ml n-Pentan bei − 5 °C 10 Minuten gerührt, abgesaugt und mit 50 ml kaltem Pentan gewaschen. Man erhält 195 g reines (78 % d. Th.) 1-Brom-2-(2-naphtyloxy)-ethan vom Schmelzpunkt 93-95 °C.

b) Herstellung des Endprodukts

Die Lösung von 15,3 g (0,06 Mol) 1-Brom-2-(2-naphthyloxy)-ethan und 7 g (0,07 Mol) N-Methylpiperidin in 30 ml Dioxan und 30 ml Acetonitril wird 16 Stunden bei 70 °C gerührt. Nach Abkühlen auf +10 °C wird der gebildete, weiße Niederschlag abgesaugt, mit 30 ml Ether und schließlich mit 50 ml Pentan gewaschen und getrocknet. Man erhält 13,6 g (64,8 % d. Th.) N-Methyl-N-2-(2-naphthyloxy)-ethylpiperidiniumbromid vom Schmelzpunkt 179-181 °C.

Analog Beispiel 1 und 2 wurden folgende Verbindungen der Formel I hergestellt, deren Strukturen durch $^{1}$H-Kernresonanz und IR-Spektren gesichert wurden :

3. 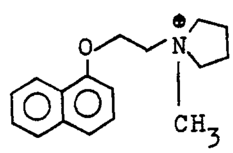 Br$^{\ominus}$ Fp. 165-168°C

4. 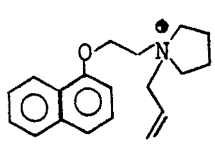 Br$^{\ominus}$ Fp. 96-99°C

5. 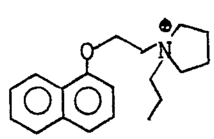 Br$^{\ominus}$ Fp. 174-176°C

6. 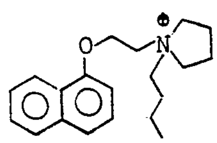 Br$^{\ominus}$ Fp. 113-175°C

| | | | |
|---|---|---|---|
| 7. | 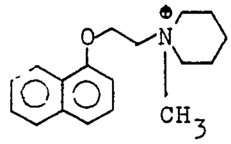 | $Br^{\ominus}$ | Fp. 158-160°C |
| 8. | 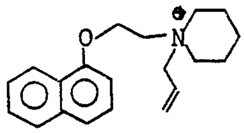 | $Br^{\ominus}$ | Fp. 183-185°C |
| 9. | 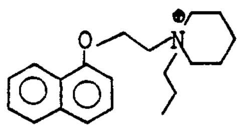 | $Br^{\ominus}$ | Fp. 178-180°C |
| 10. | 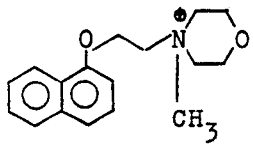 | $Br^{\ominus}$ | Fp. 172-175°C |
| 11. | 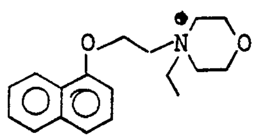 | $Br^{\ominus}$ | Fp. 164-166°C |
| 12. | 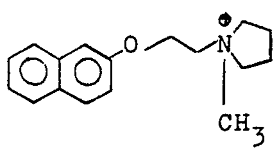 | $Br^{\ominus}$ | Fp. 173-175°C |
| 13. | 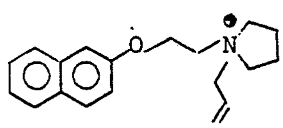 | $Br^{\ominus}$ | Fp. 123-126°C |
| 14. | 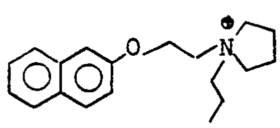 | $Br^{\ominus}$ | Fp. 125-127°C |
| 15. | 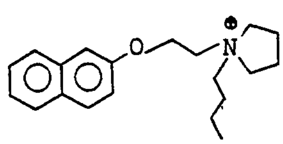 | $Br^{\ominus}$ | Fp. 117-118°C |
| 16. | 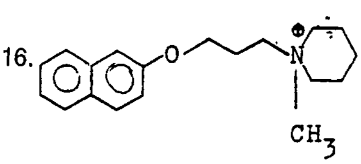 | $Br^{\ominus}$ | Fp. 154-156°C |

17. 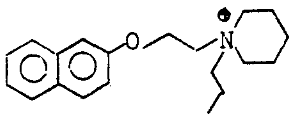 $Br^{\ominus}$ Fp. 194-196°C

18. 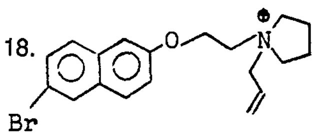 $Br^{\ominus}$ Fp. 118-121°C

19. 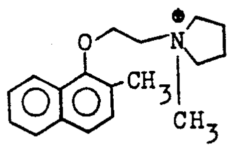 $Br^{\ominus}$ Fp. 123-126°C

20. 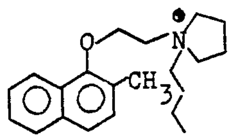 $Br^{\ominus}$ Fp. 145-148°C

21. 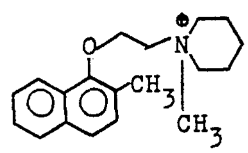 $Br^{\ominus}$ Fp. 158-160°C

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach den bisherigen Erfahrungen, daß ein Wirkstoff eine oder auch mehrere verschiedenartige Wirkungen auf Pflanzen ausüben kann.

Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren (Samenbeize, Bodenbehandlung oder Blattapplikation),

d) von den geoklimatischen Faktoren, z. B. Sonnenscheindauer, Durchschnittstemperatur, Niederschlagsmenge,

e) von der Bodenbeschaffenheit (einschließlich Düngung),

f) von der Formulierung bzw. Anwendungsform des Wirkstoffs und schließlich

g) von den angewendeten Konzentrationen der aktiven Substanz.

In jedem Falle sollen Wachstumsregulatoren die Kulturpflanzen in gewünschter Weise positiv beeinflussen.

Aus der Reihe der verschiedenartigen Anwendungsmöglichkeiten der erfindungsgemäßen Pflanzenwachstumsregulatoren im Pflanzenanbau, in der Landwirtschaft und im Gartenbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf ; außerdem ist eine dunklere Blattfärbung zu beobachten.

Als vorteilhaft für die Praxis erweist sich z. B. die Verringerung des Grasbewuchses an Straßenrändern, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des « Lagerns » (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung von Wachstumsregulatoren kann auch die seitliche Verzweigung der Pflanzen

vermehrt oder gehemmt werden. Daran besteht Interesse, wenn z. B. bei Tabakpflanzen die Ausbildung von Seitentrieben (Geiztrieben) zugunsten des Blattwachstums gehemmt werden soll.

Ein weiterer Mechanismus der Ertragssteigerung mit Wachstumshemmern beruht darauf, daß die Nährstoffe in stärkerem Maße der Blüten- und Fruchtbildung zugute kommen, während das vegetative Wachstum eingeschränkt wird. Ferner kann so wegen der relativ geringen Blatt- bzw. Pflanzenmasse dem Befall mit verschiedenen, insbesondere pilzlichen Krankheiten vorgebeugt werden.

Die Hemmung des vegetativen Wachstums ermöglicht außerdem bei vielen Kulturpflanzen eine dichtere Bepflanzung, so daß ein Mehrertrag bezogen auf die Bodenfläche erzielt werden kann. Die erfindungsgemäßen Verbindungen eignen sich besonders zur Hemmung des vegetativen Wachstums bei Kulturpflanzen wie Soja, Sonnenblumen, Erdnüssen, Raps, Zierpflanzen, Baumwolle, Reis und Gräsern.

B. Mit den neuen Wirkstoffen lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die neuen Stoffe Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Wachstumsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt und Sproßteil der Pflanze ist auch für ein gut kontrollierbares Entblättern der Bäume wesentlich.

Die Wirkung der neuen Verbindungen ist besser als bei bekannten Wachstumsregulatoren. Die Wirkung zeigt sich sowohl bei Monokotylen, z. B. Getreide wie Weizen, Gerste, Roggen, Hafer und Reis oder Mais oder Gräsern als auch insbesondere bei Dikotyledonen (z. B. Sonnenblumen, Tomaten, Erdnüssen, Reben, Baumwolle, Raps und vor allem Soja) und verschiedenen Zierpflanzen wie Chrysanthemen, Poinsettien und Hibiskus.

Die erfindungsgemäßen Mittel können den Kulturpflanzen sowohl vom Samen her (als Saatgutbeizmittel) als auch über den Boden, d. h. durch die Wurzel sowie — besonders bevorzugt — durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g, benötigt.

Für die Blatt- und Bodenbehandlung sind im allgemeinen Gaben von 0,001 bis 12 kg/ha bevorzugt 0, 01 bis 3 kg/ha als ausreichend zu betrachten.

Die erfindungsgemäßen Mittel können in Form üblicher Formulierungen angewendet werden wie Lösungen, Emulsionen, Suspensionen, Stäube, Pulver, Pasten und Granulate. Die Anwendungsformen richten sich ganz nach den Verwendungszwecken ; sie sollen in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel zugesetzt werden können. Als Hilfsstoffe zur Formulierung kommen im wesentlichen in Frage Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Amine (z. B. Ethanolamin), Dimethylformamid und Wasser ; feste Trägerstoffe wie natürliche Gesteinsmehle (z. B. Kaoline, Tonerden, Talkum, Kreide) und synthetische Gesteinsmehle (z. B. hochdisperse Kieselsäure, Silikate) ; Emulgiermittel oder sonstige oberflächenaktive Mittel, wie nichtionogene und anionische Emulgatoren (z. B. Polyoxyethylen-Fettalkohol-Ether, Alkylsulfonate) und Dispergiermittel wie Lignin, Sulfitablaugen und Methylcellulose. Bevorzugt ist die Anwendung der erfindungsgemäßen Verbindungen in wäßriger Lösung gegebenenfalls unter Zusatz von mit Wasser mischbaren organischen Lösungsmitteln wie Methanol oder anderen niederen Alkoholen, Aceton, Dimethylformamid oder N-Methylpyrrolidin. Die Formulierungen enthalten im allgemeinen zwischen 0,1 und 95 Gewichsprozent Wirkstoff, vorzugsweise zwischen 0,5 und 90.

Die Formulierungen bzw. die daraus hergestellten gebrauchsfertigen Zubereitungen wie Lösungen, Emulsionen Suspensionen, Pulver, Stäube, Pasten oder Granulate werden in bekannter Weise angewendet, beispielsweise im Vorauflaufverfahren, im Nachauflaufverfahren oder als Beizmittel.

Beispiele für Formulierungen sind :

I. 20 Gewichtsteile der Verbindung des Beispiels 1 werden in 3 Gewichtsteilen des Natriumsalzes der Diisobutylnaphthalin-sulfonsäure, 17 Gewichtsteilen des Natriumsalzes einer Ligninsulfonsäure aus einer

Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

II. 3 Gewichtsteile der Verbindung des Beispiels 4 werden mit 97 Gewichtsteilen feinteiligem Kaolin innig vermischt. Man erhält auf diese Weise ein Stäubemittel, das 3 Gew.% des Wirkstoffs enthält.

III. 30 Gewichtsteile der Verbindung des Beispiels 5 werden mit einer Mischung aus 92 Gewichtsteilen pulverförmigem Kieselsäuregel und 8 Gewichtsteilen Parafinöl, das auf die Oberfläche dieses Kieselsäuregels gesprüht wurde, innig vermischt. Man erhält auf diese Weise eine Aufbereitung des Wirkstoffs mit guter Haftfähigkeit.

IV. 40 Gewichtsteile der Verbindung des Beispiels 4 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.% Wirkstoff enthält.

V. 20 Teile der Verbindung des Beispiels 6 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

VI. Man vermischt 90 Gewichtsteile der Verbindung des Beispiels mit 10 Gewichtsteilen N-Methyl-α-pyrrolidon und erhält eine Lösung, die zur Anwendung in Form kleinster Tropfen geeignet ist.

VII. 20 Gewichtsteile der Verbindung des Beispiels 8 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

VIII. 20 Gewichtsteile der Verbindung des Beispiels 16 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IX. 10 Gewichtsteile der Verbindung des Beispiels 17, 20 Gewichtsteile Polyoxyethylensorbitanmonolaurat ($^{R}$Tween 20), 20 Gewichtsteile Methanol und 50 Gewichtsteile Wasser werden zu einer Lösung verrührt, die 10 Gew.% des Wirkstoffs enthält. Durch Hinzufügen von weiterem Wasser können stärker verdünnte Lösungen hergestellt werden.

Die erfindungsgemäßen Mittel können in diesen Aufwendungsformen auch zusammen mit anderen Wirkstoffen vorliegen, wie z. B. Herbiziden, Insektiziden, Wachstumsregulatoren und Fungiziden oder auch mit Düngemitteln vermischt und ausgebracht werden. Beim Vermischen mit Wachstumsregulatoren erhält man dabei in vielen Fällen eine Vergrößerung des Wirkungsspektrums. Bei einer Anzahl solcher Wachstumsregulatormischungen treten auch synergistische Effekte auf, d. h. die Wirksamkeit des Kombinationsproduktes ist größer als die addierten Wirksamkeiten der Einzelkomponenten.

Fungizide, die mit den erfindungsgemäßen Mitteln kombiniert werden können, sind beispielsweise Dithiocarbamate und deren Derivate, wie

Ferridimethyldithiocarbamat,
Zinkdimethyldithiocarbamat,
Manganethylenbisthiocarbamat,
Mangan-Zink-ethylendiamin-bis-dithiocarbamat,
Zinkethylenbisthiocarbamat,
Tetramethylthiuramdisulfide,
Ammoniak-Komplex von Zink-(N,N-ethylen-bis-dithiocarbamat) und
N,N'-Polyethylen-bis-(thiocarbamoyl)-disulfid,
Zink-(N,N'-propylen-bis-dithiocarbamat),
Ammoniak-Komplex von Zinn-(N,N'-propylen-bis-dithiocarbamat) und
N,N'-Polypropylen-bis-(thiocarbamoyl)-disulfid ;

Nitrophenolderivate, wie

Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat ;

heterocyclische Strukturen, wie
N-Trichlormethylthio-tetrahydrophthalimid,
N-Trichlormethylthio-phthalimid,
2-Heptadecyl-2-imidazol-acetat,
2,4-Dichlor-6-(o-chloranilino)-s-triazin,
0,0-Diethyl-phthalimidophosphonthionat,
5-Amino-1-(bis-(dimethylamino)-phosphinyl)-3-phenyl-1,2,4-triazol,
5-Ethoxy-3-trichlormethyl-1,2,4-thiadiazol,
2,3-Dicyano-1,4-dithiaanthrachinon,
2-Thio-1,3-dithio-(4,5-b)-chinoxalin,
1-Butylcarbamoyl-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thiol-1-oxid,
8-Hydrochinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-2))-benzimidazol,
Piperazin-1,4-diyl-bis-1-(2,2,2-trichlor-ethyl)-formamid,
2-Thiazolyl-(4))-benzimidazol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

und verschiedene Fungizide, wie
Dodecylguanidinacetat,
3-(2-(3,5-Dimethyl-2-oxycyclohexyl)-2-hydroxyethyl)-glutarimid,
Hexachlorbenzol,
N-Dichlorfluormethylthio-N,N'-dimethyl-N-phenyl-schwefelsäurediamid,
D,L-Methyl-N-(2,6-dimethyl-phenyl)-N-furyl(2)-alaninat,
D,L-N-(2,6-Dimethyl-phenyl)-N-(2'-methoxyacetyl)-alaninmethylester,
5-Nitro-isophthalsäure-di-isopropylester,
2,5-Dimethyl-furan-3-carbonsäureanilid,
2,5-Dimethyl-furan-3-carbonsäure-cyclohexylamid,
2-Methyl-benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,
2,3-Dichlor-1,4-naphthochinon,
1,4-Dichlor-2,5-dimethoxybenzol,
p-Dimethylaminobenzol-diazinnatriumsulfonat,
1-Chlor-2-nitro-propan,

Polychlornitrobenzole, wie Pentachlornitrobenzol, Methylisocyanat, fungizide Antibiotika, wie Griseofulvin oder Kasugamycin, Tetrafluordichloraceton, 1-Phenylthiosemicarbazid, Bordeauxmischung, nickelhaltige Verbindungen und Schwefel.

In dem nachfolgenden Beispiel A wird die Wirkung der erfindungsgemäß verwendbaren Stoffe als Pflanzenwachstumsregulatoren dargestellt, ohne die Möglichkeit weiterer Anwendungen als Wachstumsregulatoren auszuschließen.

Beispiel A, Gewächshausversuch

Zur Bestimmung der wachstumsregulierenden Eigenschaft der Prüfsubstanzen wurden Testpflanzen auf ausreichend mit Nährstoffen versorgtem Torfkultursubstrat in Kunststoffgefäßen von ca. 12,5 cm Durchmesser angezogen.

Im Nachauflaufverfahren wurden die zu prüfenden Substanzen in wäßriger Aufbereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleichssubstanz diente CCC :

$$CH_3-\overset{CH_3}{\underset{CH_3}{N^{\oplus}}}-CH_2-CH_2-Cl \qquad Cl^{\ominus}$$

Gleichlautend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die Einzeldaten sind der folgenden Tabelle zu entnehmen.

Tabelle

Beeinflussung des Längenwachstums von Sojabohnen « SRF 450 »
Nachauflaufverfahren ; Versuchsdauer : 25 Tage

| Substanz des Beispiels | Konz. mg Wirksubstanz/Gefäß | Wuchshöhe cm | % |
|---|---|---|---|
| - | - | 24,8 | 100 |
| CCC | 1,5 | 21,5 | 86,7 |
| | 6 | 19,5 | 78,6 |
| 1 | 1,5 | 18,0 | 72,6 |
| | 6 | 18,0 | 72,6 |
| 4 | 1,5 | 18,0 | 72,6 |
| | 6 | 16,5 | 66,5 |
| 5 | 1,5 | 17,5 | 70,6 |
| | 6 | 14,5 | 58,5 |
| 9 | 1,5 | 21,0 | 84,7 |
| | 6 | 19,0 | 76,6 |
| 15 | 1,5 | 20,0 | 80,7 |
| | 6 | 19,0 | 76,6 |
| 16 | 1,5 | 17,5 | 70,6 |
| | 6 | 16,0 | 64,5 |
| 17 | 1,5 | 15,0 | 60,5 |
| | 6 | 14,0 | 56,5 |

**Ansprüche**

1. Mittel zur Regulierung des Pflanzenwachstums, enthaltend ein substituiertes Alkylammoniumsalz der Formel I

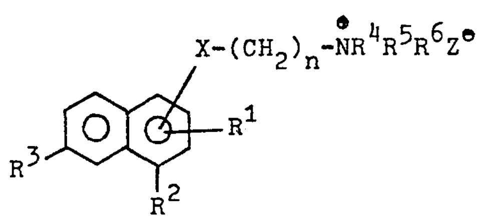

(I)

in welcher

$R^1$, $R^2$ und $R^3$ gleich oder verschieden sind und Wasserstoff, $C_{1-4}$-Alkyl oder Fluor, Chlor oder Brom bedeuten,

X Sauerstoff oder Schwefel darstellt,

n die Zahl 2, 3 oder 4 ist,

$R^4$ geradkettiges oder verzweigtes $C_{1-6}$-Alkyl, $C_{2-6}$-Alkenyl oder $C_{2-6}$-Alkinyl bedeuten, die durch Halogen, Cyan oder $C_{1-4}$-Alkoxy substituiert sein können, und

$R^5$ und $R^6$ als Alkylengruppen Teil eines gegebenenfalls durch $C_{1-2}$-Alkyl substituierten 5- oder 6-gliedrigen Heterocyclus mit 1 bis 3 Heteroatomen im Ring sind und

Z das Anion einer beliebigen nicht phytotoxischen Säure HZ bedeutet, sowie übliche Inertstoffe.

2. Mittel zur Regulierung des Pflanzenwachstums enthaltend eine Verbindung ausgewählt aus der Gruppe bestehend aus N-Propyl-N-2-(1-naphthyloxy)-ethylpyrrolidiniumbromid, N-Allyl-N-2-(1-naphthyloxy)-ethyl-pyrrolidiniumbromid, N-Allyl-N-2-(1-naphthyloxy)-ethyl-piperidiniumbromid, N-Butyl-N-2-(1-naphthyloxy)-ethyl-pyrrolidiniumbromid, N-Butyl-N-2-(2-methyl-1-naphthyloxy)-ethylpyrrolidiniumbromid, N-2-(1-Naphthyloxy)-ethylpyrrolizidiniumbromid, N-2-(2-Naphthyloxy)-ethyl-triethylammoniumbromid, N-Allyl-N-2-(2-naphthyloxy)-ethylpyrrolidiniumbromid, N-Propyl-N-2-(2-naphthyloxy)-ethylpyrrolidiniumbromid, N-Butyl-N-2-(2-naphthyloxy)-ethyl-pyrrolidiniumbromid und N-Allyl-N-2-(2-naphthyloxy)-ethyl-piperidiniumbromid.

3. Mittel zur Regulierung des Pflanzenwachstums, enthaltend eine oder mehrere Verbindungen gemäß Anspruch 1 und einen flüssigen oder festen Trägerstoff sowie gegebenenfalls eine oder mehrere oberflächenaktive Mittel.

4. Verwendung der Verbindungen der Formel I gemäß Anspruch 1 zur Regulierung des Pflanzenwachstums.

**Claims**

1. An agent for regulating plant growth, containing a substituted alkylammonium salt of the formula I

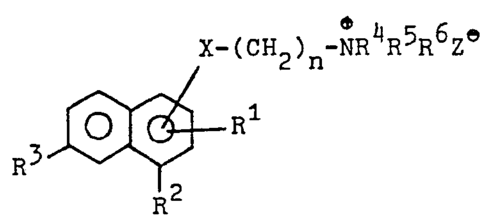

(I)

where

$R^1$, $R^2$ and $R^3$ are identical or different and each denotes hydrogen, $C_{1-4}$-alkyl, fluorine, chlorine or bromine,

X is oxygen or sulfur,

n denotes one of the integers 2, 3 and 4,

$R^4$ is linear or branched $C_{1-6}$-alkyl, $C_{2-6}$-alkenyl or $C_{2-6}$-alkynyl which are unsubstituted or substituted by halogen, cyano or $C_{1-4}$-alkoxy, and

$R^5$ and $R^6$, as alkylene groups, are part of an unsubstituted or $C_{1-2}$-alkyl-substituted 5- or 6-membered heterocycle with 1 to 3 heteroatoms in the ring, and

Z is the anion of any non-phytotoxic acid HZ, as well as usual inert substances.

2. An agent for regulating plant growth containing a compound selected from the group consisting of N-propyl-N-2-(1-naphthyloxy)-ethylpyrrolidinium bromide, N-allyl-N-2-(1-naphthyloxy)-ethylpyrrolidinium bromide, N-allyl-N-2-(1-naphthyloxy)-ethylpiperidinium bromide, N-butyl-N-2-(1-naphthyloxy)-ethylpyrrolidinium bromide, N-butyl-N-2-(2-methyl-1-naphthyloxy)-ethylpyrrolidinium bromide, N-2-(1-naphthyloxy)-ethylpyrrolizidinium bromide, N-2-(2-naphthyloxy)-ethyltriethylammonium bromide, N-allyl-N-2-(2-naphthyloxy)-ethylpyrrolidinium bromide, N-propyl-N-2-(2-naphthyloxy)-ethylpyrrolidinium bromide N-butyl-N-2-(2-naphthyloxy)-ethylpyrrolidinium bromide and N-allyl-N-2-(2-naphthyloxy)-ethylpiperidinium bromide.

3. An agent for regulating plant growth, containing one or more compounds as claimed in claim 1, a liquid or solid carrier and, if desired, one or more surfactants.

4. Use of a compound of the formula I as claimed in claim 1 for regulating plant growth.

**Revendications**

1. Moyen de régularisation de la croissance des plantes, contenant, outre des matières inertes usuelles, un sel d'alkylammonium substitué de formule I

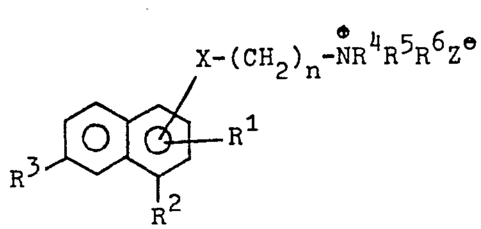

(I)

dans laquelle

$R^1$, $R^2$ et $R^3$ sont identiques ou différents et représentent hydrogène, alkyle en $C_{1-4}$ ou fluor, chlore ou brome,

X représente oxygène ou soufre,

n est le nombre 2, 3 ou 4,

$R^4$ représente alkyle en $C_{1-6}$, alcényle en $C_{2-6}$ ou alcinyle en $C_{2-6}$, à chaîne droite ou ramifiée, qui peuvent être substitués par halogène, cyano ou alcoxy en $C_{1-4}$, et

$R^5$ et $R^6$, en tant que groupes alkylène, sont partie d'un hétérocycle à 5 ou 6 chaînons et 1 à 3 hétéroatomes dans le noyau, éventuellement substitué par un alkyle en $C_{1-2}$, et.

Z représente l'anion d'un acide HZ quelconque, non phytotoxique.

2. Moyen de régularisation de la croissance des plantes contenant un composé choisi dans le groupe constitué par les bromures de N-propyl-N-2-(1-naphtyloxy)-éthylpyrrolidinium, N-allyl-N-2-(1-naphtyloxy)-éthyl-pyrrolidinium, N-allyl-N-2-(1-naphtyloxy)-éthyl-pipéridinium, N-butyl-N-2-(1-naphtyloxy)-éthyl-pyrrolidinium, N-butyl-N-2-(2-méthyl-1-naphtyloxy)-éthyl-pyrrolidinium, N-2-(1-naphtyloxy)-éthylpyrrolizidinium, N-2-(2-naphtyloxy)-éthyl-triéthylammonium, N-allyl-N-2-(2-naphtyloxy)-éthyl-pyrrolidinium, N-propyl-N-2-(2-naphtyloxy)-éthylpyrrolidinium, N-butyl-N-2-(2-naphtyloxy)-éthyl-pyrrolidinium et N-allyl-N-2-(2-naphtyloxy)-éthyl-pipéridinium.

3. Moyen de régularisation de la croissance des plantes, contenant un ou plusieurs composés selon la revendication 1 et un support liquide ou solide ainsi éventuellement qu'un ou plusieurs agents surfactifs.

4. Utilisation des composés de formule I selon la revendication 1, pour la régularisation de la croissance des plantes.